# EUROPEAN PATENT SPECIFICATION

(11) **EP 1 475 093 B1**
(45) Date of publication and mention of the grant of the patent: **06.09.2006**
(21) Application number: 04010651.0
(22) Date of filing: 24.04.2001
(51) Int. Cl.: A61K 31/44, C07D 471/02, A61P 43/00, C07D 471/04, C07D 235/00, C07D 221/00

(54) **Micronized Zolpidem hemitartrate**
Mikronisierter zolpidem Hemitartrat
Zolpidem Hemitartrate micronisé

(30) Priority: 24.04.2000 US 199298 P; 22.05.2000 US 206025 P; 14.08.2000 US 225364 P
(43) Date of publication of application: 10.11.2004
(62) Divisional of application: 01930705.7
(73) Proprietor: TEVA PHARMACEUTICAL INDUSTRIES LTD., 49131 Petah Tiqva (IL)
(72) Inventor: Aronhime, Judith, Rechovot (IL); Leonov, David, Rechovot (IL); Meszaros-Sos, Erzebet, 4031 Debrecen (HU); Salyi, Szaboles, 4031 Debrecen (HU); Zavurov, Shlomo, Lod (IL); Szabo, Csaba, 4031 Debrecen (HU)
(74) Representative: Gallagher, Kirk James

(56) References cited:
- EP-A- 0 251 859
- WO-A-00/58310
- THRELFALL ET AL: "Analysis of Organic Polymorphs" ANALYST, LONDON, GB, vol. 120, October 1995 (1995-10), pages 2435-2460, XP002101807
- LEUSEN F J J: "Ab initio prediction of polymorphs" JOURNAL OF CRYSTAL GROWTH, NORTH-HOLLAND PUBLISHING CO. AMSTERDAM, NL, vol. 166, no. 1, 1 September 1996 (1996-09-01), pages 900-903, XP004053471 ISSN: 0022-0248
- KORNBLUM S.S. ET AL: 'DISSOLUTION OF POORLY WATER-SOLUBLE DRUGS I: SOME PHYSICAL PARAMETERS RELATED TO METHOD OF MICRONIZATION AND TABLET MANUFACTURE OF A QUINAZOLINONE COMPOUND' J. OF PHARMACEUTICAL SCIENCES vol. 59, no. 5, May 1970, XP009048229
- FINCHER J.H.: 'Particle Size of Drugs and Its Relationship to Absorption and Activity' PHARMACEUTICAL SCIENCES vol. 57, no. 11, November 1968,

## Description

### FIELD OF THE INVENTION

The present invention relates to zolpidem hemitartrate Form A.

### BACKGROUND OF THE INVENTION

Zolpidem, as a hemitartrate salt, is currently approved for the short-term treatment of insomnia in the United States under the trademark of AMBIEN. Zolpidem hemitartrate is classified as a non-benzodiazepine hypnotic of the imidazopyridine class. It has little effect on the stages of sleep in normal human subjects and is as effective as benzodiazepines in shortening sleep latency and prolonging total sleep time in patients with insomnia. The development of tolerance and physical dependence for patients using AMBIEN has been seen only very rarely and under unusual circumstances. (Goodman and Gilman's, *The Pharmacological Basis of Therapeutics* 371 (Joel G. Hardman et al., eds. 9th ed. 1996)).

Zolpidem hemitartrate (CAS Registry No. 99294-93-6) has the chemical name imidazo[1,2-a]pyridine-3-acetamide,N,N,6-trimethyl-2-(4-methylphenyl)-,(2R,3R)-2,3-di hydroxy-butanedioate and is represented by the structural formula.

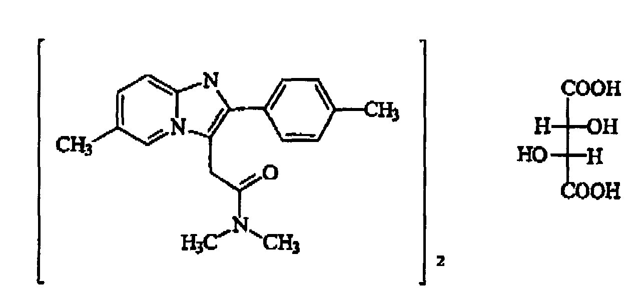

Zolpidem is among the compounds described in the following U.S. patents which are incorporated herein by reference: 4,382,938; 4,794,185; 4,356,283; 4,460,592; 4,501,745; 4,675,323; 4,808,594; and 4,847,263. The above US Patents disclose Zolpidem as having, inter alia, anxiolytic, sleep-inducing, hypnotic and anticonvulsant properties.

### SUMMARY OF THE INVENTION

The present invention provides a micronized zolpidem hemitartrate Form A having particles up to about 200 microns in size as measured by laser diffraction and an x-ray diffraction pattern having a peak at about 8.6 ±0.2 degrees two-theta.

In a preferred embodiment the zolpidem hemitartrate of the present invention is further characterized by an x-ray diffraction pattern having peaks at 6.7, 11.2, 15.4 and 17.3 ±0.2 degrees two-theta.

In a preferred embodiment the zolpidem hemitartrate Form A of the invention has particles up to about 50 microns in size as measured by laser diffraction.

In a further aspect the present invention provides a pharmaceutical composition comprising a therapeutically effective amount of zolpidem hemitartrate Form A of the present invention and a pharmaceutically acceptable carrier.

### BRIEF DESCRIPTION OF THE FIGURES

Fig. 1. shows the X-ray diffraction pattern of zolpidem hemitartrate Form A.
Fig. 2. shows the DTG thermal profile of zolpidem hemitartrate Form A.

Fig. 16. shows an X-Ray diffraction pattern for micronized Form A.

### DETAILED DESCRIPTION OF THE INVENTION

The present invention provides a new crystal form of zolpidem hemitartrate. Crystal forms of a compound can be distinguished in a laboratory by X-ray diffraction spectroscopy and by other methods such as, infrared spectrometry. It is desirable to investigate all solid state forms of a drug, including all crystal/polymorphic forms, and to determine the stability, dissolution and flow properties of each crystal/polymorphic form. For a general review of polymorphs and the pharmaceutics applications of polymorphs see G.M. Wall, *Pharm Manuf.* 3, 33 (1986); J.K. Haleblian and W. McCrone, *J. Pharm. Sci*., **58**, 911 (1969); and J.K. Haleblian, *J. Pharm. Sci*., **64**, 1269 (1975), all ofwhich are incorporated herein by reference.

As used herein, the term "zolpidem hemitartrate" includes hydrates and solvates of zolpidem hemitartrate. The term "water content" refers to the content of water based upon the Loss on Drying method (the "LOD" method) as described in Pharmacopeial Forum, Vol. 24, No. 1, p. 5438 (Jan - Feb 1998), the Karl Fisher assay for determining water content or thermogravimetric analysis (TGA). The term "equivalents of water" means molar equivalents of water. All percentages herein are by weight unless otherwise indicated. Those skilled in the art will also understand that the term "anhydrous" when used in reference to zolpidem hemitartrate describes zolpidem hemitartrate which is substantially free of water. Those skilled in the art will appreciate that the term "monohydrate" when used in reference to zolpidem hemitartrate describes a crystalline material having a water content of about 2.3% w/w. One skilled in the art will also appreciate that the term "dihydrate" when used in reference to zolpidem hemitartrate describes a crystalline material having a water content of about 4.5% w/w. One skilled in the art will also appreciate that the term "trihydrate" when used in reference to zolpidem hemitartrate describes a crystalline material having a water content of about 6.6% w/w. One skilled in the art will also appreciate that the term "tetrahydrate" when used in reference to zolpidem hemitartrate describes a crystalline material having a water content of about 8.6% w/w. One skilled in the art will also appreciate that the term "methanolate","ethanolate", "solvates of isopropranol", "solvates of butanol" or "solvates of ethylacetate" refers to Zolpidem hemitartrate in which solvent is contained within the crystal lattice in quantities above 1%. One skilled in the art will also appreciate that the term "hemiethanolate" when used in reference to zolpidem hemitartrate describes a crystalline material having an ethanol content of about 2.9% w/w.

Hydrate and solvate forms of zolpidem hemitartrate are novel and distinct from each other in terms of their characteristic powder X-ray diffraction patterns and their thermal profiles.

For the purposes of this specification, ambient temperature is from about 20°C to about 25°C.

All powder X-ray diffraction patterns were obtained by methods known in the art using a Scintag X'TRA X-ray powder diffractometer, equipped with a solid stae Si(Li) detector thermoelectrically cooled, at scanning speed of 3° min.⁻¹ scanning range 2-40 degrees two-theta. Copper radiation of λ = 1.5418 Å was used.

Measurement of thermal analysis are conducted for the purpose of evaluating the physical and chemical changes that may take place in a heated sample. Thermal reactions can be endothermic (eg, melting, boiling, sublimation, vaporization, desolvation, solid-solid phase transitions, chemical degradation, etc.) or exothermic (eg, crystallization, oxidative decomposition, etc.) in nature. Such methodology has gained widespread use in the pharmaceutical industry in characterization of polymorphism. The quantitative applications of thermal applications of thermal analysis have proven to be useful in characterization of polymorphic systems. The most commonly applied techniques are thermogravimetry (TGA), differential thermal analysis (DTA), and differential scanning calorimetry (DSC).

The DTA and TGA curves presented herein were obtained by methods known in the art using a DTG Shimadzu model DTG-50 (combined TGA and DTA). The weight of the samples was about 9 to about 13 mg. The samples were scanned up to about 300°C or above at a rate of 10°C/min. Samples were purged with nitrogen gas at a flow rate of 20 ml/min. Standard alumina crucibles covered lids with one hole.

Thermogravimetry analysis (TGA) is a measure of the thermally induced weight loss of a material as a function of the applied temperature. TGA is restricted to transitions that involve either a gain or a loss of mass, and it is most commonly used to study desolvation processes and compound decomposition.

Karl Fisher analysis, which is well known in the art, is also used to determine the quantity of water in a sample.

As used herein a slurry refers to a suspension of insoluble particles or slightly soluble particles in an aqueous or organic (non-aqueous) liquid, without complete dissolution of the solid.

### Zolpidem hemitartrate Form A

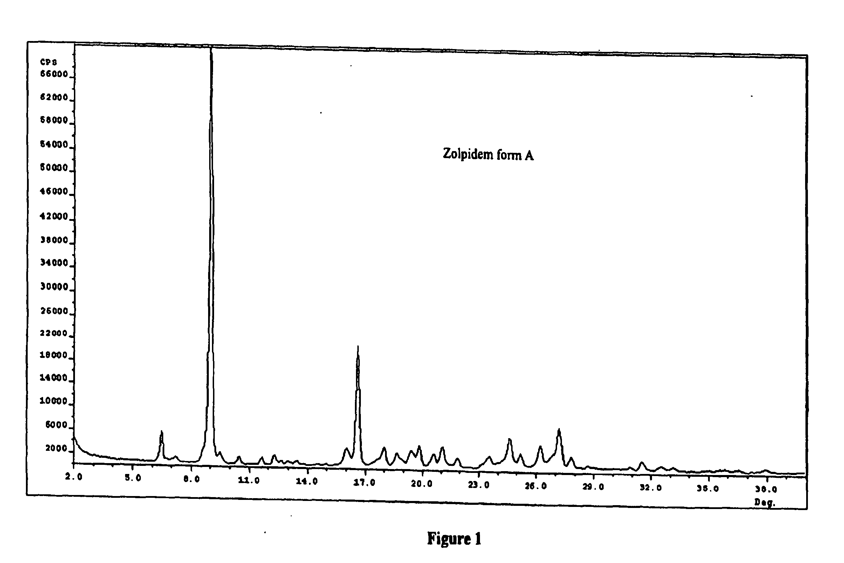

Zolpidem hemitartrate Form A ("Form A") is characterized by an X-ray diffraction pattern with peaks at about 6.5, 9.0, 16.1, 16.6, 24.6 and 27.3 ±0.2 degrees two-theta. The diffraction pattern is reproduced in Fig. 1. The above x-ray diffraction pattern was found in the prior art EP standard sample. When samples of From A containing substantially particles smaller than all 200 microns were examined the x-ray diffraction pattern showed a new peaks at about 8.6 ±0.2 degrees two-theta. Other unexpected peaks were observed at 6.7, 11.2, 15.4 and 17.3 ±0.2 degrees two-theta.

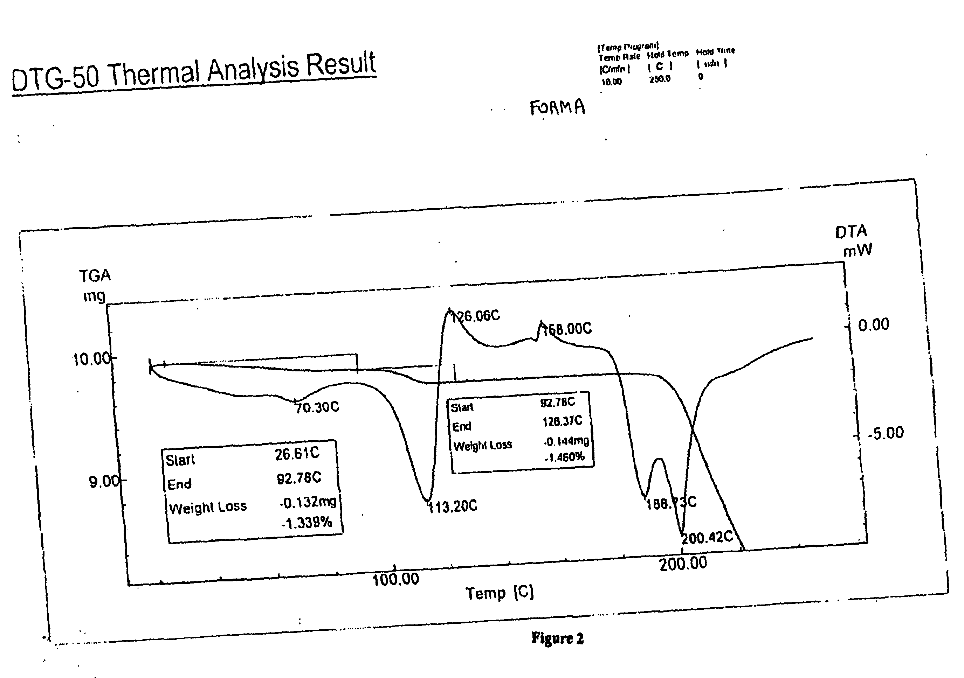

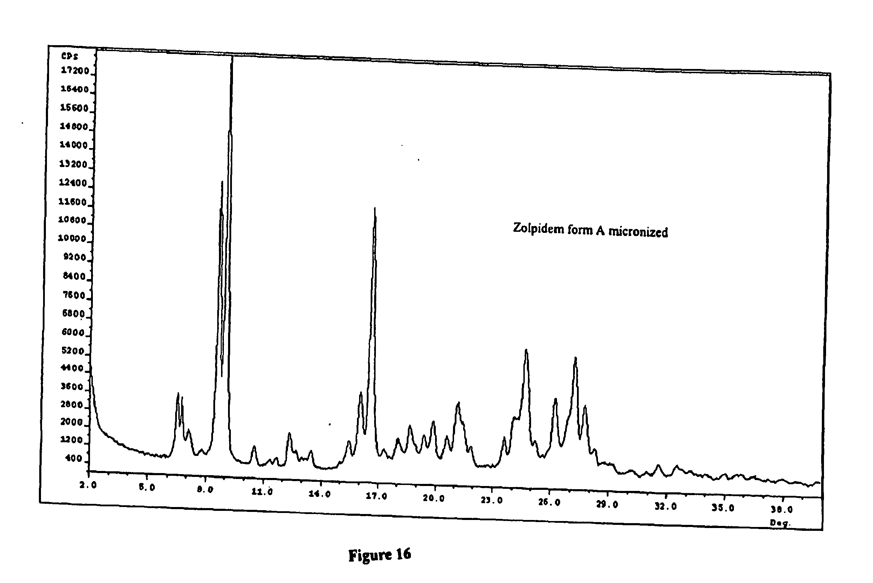

The DTG thermal profile of Form A is shown in Fig. 2. The thermal profile shows an endotherm at about 110°C, followed by an exotherm; an additional exothermic/endothermic event at above about 150°C; a melting endotherm at about 188°C; and an endothermic event at about 200°C concomitant to decomposition.

The hydration states of Form A is characterized by TGA and Karl Fisher analysis. Zolpidem hemitartrate described in the EP monograph (2001), herein identified as Form A, is reported as a hygroscopic solid. It was found by us, that Form A may contain about 1.0 % water or more, and readily absorbs up to 3.0 % water as measured by Karl Fischer analysis. The 110°C endotherm of the TGA is attributed to partial desorption of water with an overall water content of about 3%.

### SMALL PARTICLES OF ZOLPIDEM HEMITARTRATE

The present invention provides zolpidem hemitartrate Form A having a relatively small particle size and a corresponding relatively large surface area.

It has long been recognized that when a pharmaceutical composition containing a drug which is orally administered to subjects, a dissolution step is essential for the drug to be absorbed through gastrointestinal tract. A drug may have insufficient bioavailability because of the poor solubility in the gastrointestinal tract, consequently the drug passes through the site of absorption before it completely dissolves in the fluids.

Bioavailability, particularly of slightly soluble active compounds is highly dependent on the surface area of the particles and the surface area is inversely related to the size of the compound. Thus particles having relatively small particle size have a relatively greater surface area and an increased solubility rate in gastrointestinal tract.

Small zolpidem hemitartrate particles can be achieved using methods well known in the art. (See US Patents Nos. 4,151,273; 4,196,188; 4,302,446; 4,332, 721; 4,840,799; and 5,271,944, incorporated herein by reference.) Micronization as provided in Example ? in one method of generating small zolpidem hemitartrate particles. Particle size was measured by a laser diffraction instrument (Malvem Mastersizer S). The sample was analysed after proper dispersion in a solution of dioctylsulfosuccinate sodium salt in hexane (0.02% w/w).

In one embodiment, the invention provides zolpidem hemitartrate in which substantially all zolpidem hemitartrate particles have a particle size of up to about 200 micrometer. It will be understood by those of skill in the art that this embodiment includes pharmaceutical compositions containing a therapeutically effective amount of zolpidem hemitartrate.

According to another embodiment, the present invention provides zolpidem hemitartrate particles in which substantially all zolpidem hemitartrate particles, have a particle size of up to about 50 microns. It will be understood by those of skill in the art that this embodiment includes pharmaceutical compositions containing a therapeutically effective amount of zolpidem hemitartrate.

### A PHARMACEUTICAL COMPOSITION CONTAINING ZOLPIDEM MENUTARMATE

According to another aspect, the present invention relates to a pharmaceutical composition comprising zolpidem hemitartrate disclosed herein and at least one pharmaceutically acceptable excipient. Such pharmaceutical compositions may be administered to a mammalian patient in a dosage form.

The dosage forms may contain one or more of the novel forms of zolpidem hemitartrate or, alternatively, may contain one or more of the novel forms of zolpidem hemitartrate as part of a composition. Whether administered in pure form or in a composition, the zolpidem hemitartrate form(s) may be in the form of a powder, granules, aggregates or any other solid form. The compositions of the present invention include compositions for tableting. Tableting compositions may have few or many components depending upon the tableting method used, the release rate desired and other factors. For example, compositions of the present invention may contain diluents such as cellulose-derived materials like powdered cellulose, microcrystalline cellulose, microfine cellulose, methyl cellulose, ethyl cellulose, hydroxyethyl cellulose, hydroxypropyl cellulose, hydroxypropylmethyl cellulose, carboxymethyl cellulose salts and other substituted and unsubstituted celluloses; starch; pregelatinized starch; inorganic diluents such calcium carbonate and calcium diphosphate and other diluents known to one of ordinary skill in the art. Yet other suitable diluents include waxes, sugars (*e*.*g*. lactose) and sugar alcohols like mannitol and sorbitol, acrylate polymers and copolymers, as well as pectin, dextrin and gelatin.

Other excipients contemplated by the present invention include binders, such as acacia gum, pregelatinized starch, sodium alginate, glucose and other binders used in wet and dry granulation and direct compression tableting processes; disintegrants such as sodium starch glycolate, crospovidone, low-substituted hydroxypropyl cellulose and others; lubricants like magnesium and calcium stearate and sodium stearyl fumarate; flavorings; sweeteners; preservatives; pharmaceutically acceptable dyes and glidants such as silicon dioxide.

Dosage forms may be adapted for administration to the patient by oral, buccal, parenteral, ophthalmic, rectal and transdermal routes. Oral dosage forms include tablets, pills, capsules, troches, sachets, suspensions, powders, lozenges, elixirs and the like. The novel forms of zolpidem hemitartrate disclosed herein also may be administered as suppositories, ophthalmic ointments and suspensions, and parenteral suspensions, which are administered by other routes. The most preferred route of administration of the zolpidem hemitartrate forms of the present invention is oral.

Capsule dosages will contain the solid composition within a capsule which may be coated with gelatin. Tablets and powders may also be coated with an enteric coating. The enteric-coated powder forms may have coatings comprising phthalic acid cellulose acetate, hydroxypropylmethyl cellulose phthalate, polyvinyl alcohol phthalate, carboxymethylethylcellulose, a copolymer of styrene and maleic acid, a copolymer of methacrylic acid and methyl methacrylate, and like materials, and if desired, they may be employed with suitable plasticizers and/or extending agents. A coated tablet may have a coating on the surface of the tablet or may be a tablet comprising a powder or granules with an enteric-coating.

The currently marketed form of zolpidem hemitartrate (AMBIEN) are a 5 and a 10 mg tablet which includes the following inactive ingredients: hydroxypropyl methylcellulose, lactose, magnesium stearate, microcrystalline cellulose, polyethylene glycol, sodium starch glycolate, titanium dioxide; the 5 mg tablet also contains FD&C Red No. 40, iron oxide colorant, and polysorbate 80.

The function and advantage of these and other embodiments of the present invention will be more fully understood from the examples below. The following examples are intended to illustrate the benefits of the present invention, but do not exemplify the full scope of the invention.

### EXAMPLES

### EXAMPLE 1

### Preparation of zolpidem hemitartrate Form A by crystallization,

Crude zolpidem hemitartrate (6.1 g) is suspended in 90 ml of methanol and the mixture solution is heated to 44-46°C. The solution is agitated at this temperature for 30 minutes. The 6.1 g crude salt is dissolved after 30 minutes agitating at this temperature. The clear mixture solution is cooled to room temperature and stirred for 3 hours. The methanol is evaporated in vacuum to a mixture solution volume of 12 ml. The resulting mixture solution is cooled and kept for 12 hours at 0-5°C, and then filtered. The crystalline product is dried under vacuum (70 to 100 mbar) at 38°C for 12 hours.

### EXAMPLE 2

### Micronization of zolpidem hemitartrate

Pure dry zolpidem hemitartrate was micronized in an air jet micronizer (CHRISPRO Jetmill MC-20OKX, BD). The feeding rate was set at 9.0 kg/hr. The feeding air pressure was set at 6.0 bar. The grinding air pressure was set 3.5 bar. The particle size of the micronized zolpidem hemitartrate was found to be less than 20 microns Malvern laser diffraction Mastersizer S.

### EXAMPLE 3

### X-Ray powder diffraction spectra of micronization of zolpidem hemitartrate Form A

Zolpidem hemitartrate Form A was micronized as in Example 33 to a particle size up to 20 microns as determined by laser diffraction. The X-Ray powder diffraction spectra showed an unexpected peak at about 8.6 degrees two-theta. Other unexpected peaks were observed at 6.7, 11.2, 15.4 and 17.3 ±0.2 degrees two-theta. An X-Ray diffraction pattern for micronized Form A is shown in Fig. 16.

## Claims

1. Micronized zolpidem hemitartrate Form A having particles up to about 200 microns in size as measured by laser diffraction and an x-ray diffraction pattern having a peak at about 8.6 ±0.2 degrees two-theta.

2. The zolpidem hemitartrate of claim 1, further **characterized by** an x-ray diffraction pattern having peaks 6.7, 11.2, 15.4 and 17.3 ±0.2 degrees two-theta.

3. The zolpidem hemitartrate of claim 1 or claim 2, wherein the zolpidem hemitartrate particles are up to about 50 microns in size as measured by laser diffraction.

4. A pharmaceutical composition comprising a therapeutically effective amount of zolpidem hemitartrate according to any preceding claim and a pharmaceutically acceptable carrier.

## Patentansprüche

1. Mikronisiertes Zolpidem-Hemitartrat der Form A mit Teilchen von bis zu etwa 200 Mikrometern Größe, gemessen durch Laserdiffraktion, und einem Röntgenbeugungsdiagramm mit einem Peak bei etwa 8,6 ± 0,2 Grad Zwei-Theta.

2. Zolpidem-Hemitartrat nach Anspruch 1, welches weiterhin durch ein Röntgenbeugungsdiagramm mit Peaks bei 6,7, 11,2, 15,4 und 17,3 ± 0,2 Grad Zwei-Theta **gekennzeichnet** ist.

3. Zolpidem-Hemitartrat nach Anspruch 1 oder Anspruch 2, wobei die Zolpidem-Hemitartrat-Teilchen bis zu etwa 50 Mikrometer groß sind, gemessen durch Laserdiffraktion.

4. Pharmazeutische Zusammensetzung, die eine therapeutisch wirksame Menge an Zolpidem-Hemitartrat nach einem der vorangegangenen Ansprüche und einen pharmazeutisch verträglichen Träger enthält.

## Revendications

1. Forme A de l'hémitartrate de zolpidem micronisée, consistant en particules présentant une dimension, mesurée par diffraction laser, pouvant atteindre environ 200µ, et présentant un diagramme de diffraction des rayons X ayant un pic à un angle deux thêta d'environ 8,6 ± 0,2 degrés.

2. Hémitartrate de zolpidem selon la revendication 1, **caractérisé en outre par** un diagramme de diffraction des rayons X ayant des pics à un angle deux thêta de 6,7, 11,2, 15,4 et 17,3 ± 0,2 degrés.

3. Hémitartrate de zolpidem selon la revendication 1 ou la revendication 2, dans lequel les particules d'hémitartrate de zolpidem ont une dimension, mesurée par diffraction laser, pouvant atteindre environ 50µ.

4. Composition pharmaceutique comprenant une quantité thérapeutiquement efficace d'hémitartrate de zolpidem selon l'une quelconque des revendications précédentes et comprenant un véhicule pharmaceutiquement acceptable.
